# EUROPEAN PATENT APPLICATION

(11) **EP 4 682 245 A1**
(43) Date of publication of application: **21.01.2026**
(21) Application number: 23928366.6
(22) Date of filing: 13.11.2023
(51) Int. Cl.: C12N 5/00, C12N 5/02, A61L 27/38, C07K 14/47, C12N 5/073, C12N 5/075

(54) **METHOD FOR PREPARING MATURE OOCYTES AND USE**

(30) Priority: 17.03.2023 CN 202310270106
(71) Applicant: Shanghai Jiao Tong University, Shanghai 200240 (CN)
(72) Inventor: WU, Ji, Shanghai 200240 (CN); LI, Xiaoyong, Shanghai 200240 (CN); GE, Junbang, Shanghai 200240 (CN)
(74) Representative: Potter Clarkson
(86) International application number: PCT/CN2023/131356
(87) International publication number: WO 2024/193062

(57) **Abstract**

A method for preparing mature oocytes and a use. An ovary organ system derived from female germline stem cells (FGSCs) is utilized to efficiently differentiate the FGSCs into functional oocytes in vitro. The oocytes can be further developed by in vitro fertilization. The method can efficiently obtain functional oocytes, provide a new approach for assisted reproduction, and provide a new method for female infertility treatment.

## Description

### FIELD OF THE DISCLOSURE

The present disclosure belongs to the field of infertility and fertility preservation, and more specifically, relates to a method for preparing mature oocytes, comprising differentiating human female germline stem cells into human mature oocytes.

### BACKGROUND OF THE DISCLOSURE

In recent years, the global incidence of infertility has been increasing annually, with a rising average prevalence rate. Infertility has become the third most significant disease threatening human health, following cardiovascular diseases and cancer. Furthermore, underlying these stark data is a trend of rising infertility among younger populations, with the highest proportion found among individuals aged 25-30 years. Oocyte scarcity and declining quality are key factors leading to female infertility. The discovery of female germline stem cells (FGSCs) offers a new perspective for addressing oocyte shortage. As germline stem cells capable of replenishing oocytes, FGSCs can maintain ovarian reproductive function, indirectly support ovarian endocrine activity, and delay premature ovarian failure. They are of significant importance for improving follicle quality and pregnancy rate in female mammals and are becoming a focal point in medical research. FGSCs are a special type of adult stem cell that possess not only the characteristics of stem cells but also the ability to transmit genetic information. Through a complex developmental process, FGSCs in female mammals ultimately give rise to functional mature oocytes, providing the female gametes for offspring development.

As early as 2012, Japanese scientists Mitinori Saitou and Katsuhiko Hayashi developed methods to differentiate mouse embryonic stem cells and induced pluripotent stem cells into primordial germ cells (PGCs). However, a limitation was that these in vitro-derived PGCs needed to be transplanted into adult mouse ovaries to obtain oocytes. Moreover, due to species differences, this method is not readily applicable to humans.

In October 2016, the research team led by Katsuhiko Hayashi successfully generated oocyte-like cells from mouse skin cells, which were subsequently fertilized in vitro and developed into healthy mice. This represented the world's first successful complete in vitro generation of functional oocytes. However, since the starting material was skin cells and considering the significant species-specific differences in germ cell properties and culture requirements, this method is not easily applicable to humans.

In 2018, Mitinori Saitou's research team induced human induced pluripotent stem cells (iPSCs) into PGC-like cells (PGCLCs), then co-cultured with somatic cells. Eventually, iPSCs were induced into cells exhibiting characteristics of oocytes. However, these cells were identified as oogonia-like and could not mature or be fertilized in vitro.

In summary, there is currently no report in the art on obtaining mature human oocytes in vitro.

### SUMMARY OF THE DISCLOSURE

The purpose of the present disclosure is to provide a method for preparing mature oocytes, comprising differentiating human female germ stem cells into mature oocytes.

In the first aspect, the present disclosure provides an in vitro method for preparing human mature oocytes, wherein the method comprises: (1) co-culturing human female germline stem cells with ovarian somatic-like cells to establish a human ovarian organoid system, wherein follicles are generated within the human ovarian organoid system; and (2) isolating follicles from the human ovarian organoids to obtain human mature oocytes by in vitro culture.

In one or more embodiments, after obtaining the human mature oocytes, the method further comprises: (3) performing in vitro fertilization of the human mature oocytes, followed by in vitro development (e.g., into a blastocyst).

In one or more preferable embodiments, in (1), the ovarian somatic-like cells comprise ovarian somatic-like cells obtained by in vitro differentiation of human pluripotent stem cells.

In one or more preferable embodiments, the ovarian somatic-like cells are cultured by the following steps comprising:
(1) cell pre-culture;
(2) treating the cells of (1) with a GSK3 inhibitor;
(3) treating the cells of (2) with B/A/W, wherein the B/A/W is BMP4, ActivinA and Wnt3a;
(4) treating the cells of (3) with Follistain, BMP4 and serum;
(5) treating the cells of (4) with Follistain and BMP4 to obtain a culture comprising human ovarian somatic cell-like cells.

In one or more preferable embodiments, the human pluripotent stem cells comprise human induced pluripotent stem cells (iPSCs) and human embryonic stem cells.

In one or more preferable embodiments, in (1), the human female germline stem cells and ovarian somatic-like cells are mixed at a ratio of 1:(100-10000); preferably 1:(200-8000); more preferably 1:(300-6000).

In one or more preferable embodiments, after mixing the human female germline stem cells and ovarian somatic-like cells, the mixed cells are first cultured to form cell aggregates and then to form organoids.

In one or more preferable embodiments, the human female germline stem cells are mixed with ovarian somatic cell-like cells and then cultured in three dimensions (for example, solidified with Matrigel).

In one or more preferable embodiments, after mixing the human female germline stem cells and ovarian somatic-like cells, the mixture is cultured in a first organoid culture medium to form cell aggregates, comprising: retinoic acid, L-glutamine, spermidine, sodium pyruvate, β-mercaptoethanol, EGF, FGF, glial cell derived neurotrophic factor, leukemia inhibitory factor, non-essential amino acids; subsequently cultured in a second organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol; thereafter cultured in a third organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol.

In one or more preferable embodiments, the culture by the first organoid culture medium is lasted for 5-10 days, preferably 6-9 days (such as 7, 8 days); the culture by the second organoid culture medium is lasted for 10-20 days, preferably 12-18 days (such as 13, 14, 15, 16, 17 days); or the culture by the third organoid culture medium is lasted for 18-45 days, preferably 20-40 days (such as 22, 24, 28, 30, 32, 35, 38 days).

In one or more preferable embodiments, the first or second organoid culture medium comprises MEMα or a similar medium (such as culture medium of other MEM series) as the basal culture medium, wherein the first organoid culture medium comprises serum or a serum protein substitute (such as 5-15%, preferably 7-13%, more preferably 8-12%), and the second organoid culture medium comprises serum or a serum protein substitute (preferably 1-5%, such as 1.5%, 2%, 2.5%, 3%, 3.5%, 4% or 4.5%); the third organoid culture medium comprises StemPro-34 SFM as the basal culture medium and serum or a serum protein substitute (preferably 5-15%, such as 7%, 8%, 9%, 10%, 11%, 12% or 13%).

In one or more preferable embodiments, the human female germline stem cells are mixed with ovarian somatic cell-like cells at a ratio of 1:400, 500, 600, 800, 1000, 1500, 2000, 3000, 4000, 5000, 7000 or 9000.

In one or more preferable embodiments, during the culture, the medium is replaced (partially replaced) every 12-36 hours.

In one or more preferable embodiments, when cultured by the first organoid culture medium, the cells are cultured in a U-shaped plate.

In one or more preferable embodiments, when cultured by the second organoid culture medium, the cells are cultured in a Transwell.

In one or more preferable embodiments, when cultured by the third organoid culture medium, the cells are cultured in a Transwell.

In one or more preferable embodiments, in the first, second or third organoid culture medium, β-mercaptoethanol is used to neutralize oxygen free radicals accumulated in the culture medium.

In one or more preferable embodiments, in the first, second or third organoid culture medium, the content of β-mercaptoethanol in the first organoid culture medium is 1.5 to 3 times (such as 2 times) of that in the second or third organoid culture medium.

In one or more preferable embodiments, the first, second or third organoid culture medium further comprises antibiotics.

In one or more preferable embodiments, the antibiotics comprise: penicillin and/or streptomycin.

In one or more preferable embodiments, in step (2), the in vitro culture comprises: culturing in a first follicle culture medium comprising: L-glutamine, transferrin, sodium selenite, ascorbic acid, EGF, Activin-A, FSH, β-mercaptoethanol; subsequently culturing in a second follicle culture medium comprising: L-glutamine, sodium pyruvate, sodium selenite, bFGF, EGF, transferrin, insulin, PVP, bmp15, gdf9, cAMP, FSH, LH, Estrogen, Progesterone, β-mercaptoethanol; thereafter culturing in a third follicle culture medium comprising: the second follicle culture medium and 5-20% (v/v) inactivated human follicular fluid.

In one or more preferable embodiments, the first follicle culture medium comprises MEMα or a similar medium (such as culture medium of other MEM series) as the basal culture medium and serum or a serum protein substitute (preferably 3-8%, such as 4%, 5%, 6%, 7%); the second follicle culture medium comprises MEMα or a similar medium (such as culture medium of other MEM series) as the basal culture medium and serum or a serum protein substitute (preferably 5-15%, such as 7%, 8%, 9%, 10%, 11%, 12% or 13%).

In one or more preferable embodiments, the culture is three-dimensional culture (e.g., solidified with Matrigel).

In one or more preferable embodiments, during the culture, the medium is replaced (partially replaced) every 12-36 hours.

In one or more preferable embodiments, when cultured by the first, second or third follicle culture medium, the cells are cultured in a Transwell.

In one or more preferable embodiments, the first, second or third follicle culture medium further comprises β-mercaptoethanol.

In one or more preferable embodiments, the first, second or third follicle culture medium further comprises antibiotics.

In one or more preferable embodiments, the antibiotics comprise: penicillin and/or streptomycin.

In another aspect, the present disclosure provides a human mature oocyte prepared by any one of the above-mentioned methods.

In one or more preferable embodiments, the mature human oocytes are not able to independently develop into an organism without in vitro fertilization.

In another apect, the present disclosure provides a use of human female germline stem cells and ovarian somatic-like cells for preparing human mature oocytes in vitro.

In one or more preferable embodiments, the human mature oocytes can be further developed in vitro into a blastocyst via in vitro fertilization.

In another aspect, the present disclosure provides a kit for preparing human mature oocytes in vitro, wherein it comprises:
(a) human female germline stem cells and ovarian somatic-like cells, or a mixture thereof;
(b) an organoid culture medium; and
(c) a follicle culture medium.

In one or more preferable embodiments, in (a), the human female germline stem cells and ovarian somatic-like cells are mixed at a ratio of 1:(100-10000); preferably 1:(200-8000); more preferably 1:(300-6000).

In one or more preferable embodiments, (b) comprises: a first organoid culture medium comprising: retinoic acid, L-glutamine, spermidine, sodium pyruvate, β-mercaptoethanol, EGF, FGF, glial cell derived neurotrophic factor, leukemia inhibitory factor, non-essential amino acids; a second organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol; a third organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol; preferably, the first or second organoid culture medium comprises MEMα or a similar medium as the basal culture medium, wherein the first organoid culture medium comprises serum or a serum protein substitute (such as 5-15%, preferably 7-13%, more preferably 8-12%), and the second organoid culture medium comprises serum or a serum protein substitute (preferably 1-5%, such as 1.5%, 2%, 2.5%, 3%, 3.5%, 4% or 4.5%); the third organoid culture medium comprises StemPro-34 SFM as the basal culture medium and serum or a serum protein substitute (preferably 5-15%, such as 7%, 8%, 9%, 10%, 11%, 12% or 13%).

In one or more preferable embodiments, (c) comprises: a first follicle culture medium comprising: L-glutamine, transferrin, sodium selenite, ascorbic acid, EGF, Activin-A, FSH, β-mercaptoethanol; a second follicle culture medium comprising: L-glutamine, sodium pyruvate, sodium selenite, bFGF, EGF, transferrin, Insulin, PVP, bmp15, gdf9, cAMP, FSH, LH, Estrogen, Progesterone, β-mercaptoethanol; a third follicle culture medium comprising: the second follicle culture medium and 5-20% (v/v) inactivated human follicular fluid.

Other aspects of the present disclosure will be apparent to those skilled in the art based on the disclosure herein.

### DESCRIPTION OF DRAWINGS

Figure 1A. Bright field image of human pluripotent stem cells obtained in Example 1 differentiating into human ovarian somatic cell-like cells.
Figure 1B. Representative images of ovarian organoid cultured at the first week (W1), the third week (W3) and the fifth week (W5).
Figure 2. Results of ovarian organoid cultured at the first week (W1), the third week (W3) and the fifth week (W5) by flow cytometry analysis.
Figure 3. Distinct RFP signal observed at the fifth week of ovarian organoid culture.
Figure 4. MVH expression in germ cells of ovarian organoid cultured at the fifth week.
Figure 5. Hormone concentration of ovarian organoid culture.
Figure 6. Morphological images of follicles derived from the in vitro differentiation of human female germline stem cells at different culture stages.
Figure 7. Morphology of oocytes at GV stage differentiated in vitro from human female germline stem cells.
Figure 8. Morphology of oocytes at MII stage differentiated in vitro from human female germline stem cells.
Figure 9. Embryonic development following in vitro fertilization of mature oocytes differentiated in vitro from human female germline stem cells.
Figure 10. Immature follicles subjected to 3D culture were placed in in vitro maturation medium and cultured in a 37°C, 5% CO₂ incubator for 24-72h. Oocyte morphology and the degree of cumulus cell expansion were observed under a microscope, with the extrusion of the first polar body used as the marker for maturation.
Figure 11. Once the oocytes are mature, ICSI fertilization is performed and the appearance of pronuclei is observed after 16-24 hours. The appearance of pronuclei and bipolar bodies is regarded as a sign of fertilization.

### DETAILED DESCRIPTION

Based on in-depth researches, the inventors have established an ovarian organoid system derived from female germline stem cells (FGSCs) (preferably established using a three-dimensional (3D) culture system), and this system is utilized to efficiently differentiate female germline stem cells into functional oocytes in vitro. The oocytes can be further developed by in vitro fertilization (ultimately forming healthy offspring). The method can efficiently obtain functional oocytes, provide a new approach for assisted reproduction, and provide a new method for female infertility treatment.

### Terms

As used herein, the term "basal medium" or "basic culture medium" refers to a medium suitable for cell culture, providing sufficient nutritional components for cells to meet the requirements for cell growth.

As used herein, the terms "granulosa cells" and "ovarian granulosa cells" are used interchangeably and they may be cells of natural origin or cells expanded/passaged based on natural cells. In addition, they may also be cells that have been genetically engineered or modified.

As used herein, the terms "female germline stem cells" and "ovarian female germline stem cells" are used interchangeably and they may be cells of natural origin or cells expanded/passaged based on natural cells. In addition, they may also be cells that have been genetically engineered or modified.

As used herein, the "ovarian somatic cell-like cells" and "ovarian somatic cells" are used interchangeably and they can be cells of natural origin or cells expanded/passaged based on natural cells, or they can also be cells induced. In addition, they can also be cells that have been genetically engineered or modified.

As used herein, "functional" means that the ovarian somatic cell-like cells obtained according to the described method have the same or similar functions as expected.

As used herein, "differentiation" refers to the developmental process of lineage commitment. "Lineage" refers to the pathway of cell development wherein a precursor or "progenitor" cell (a stem cell in the present disclosure) undergoes progressive physiological process to become a specific cell type with characteristic functions (ovarian somatic-like cells in the present disclosure).

### Female Germline Stem Cells

In the present disclosure, the female germline stem cells can be female germline stem cells from the body itself, or female germline stem cells that have been expanded, cultured, passaged or established in the field. For example, the inventors have expanded and propagated female germline stem cell in previous studies and these in vitro expanded female germline stem cells can be utilized. For example, a method for culturing (expanding) female germline stem cells comprise: placing female germline stem cells in an embryonic fibroblast (STO cell) feeder layer, and culturing them in a cell culture medium comprising sodium pyruvate, L-glutamine, mercaptoethanol, non-essential amino acids, epidermal growth factor, human basic fibroblast growth factor, glial cell growth factor and leukemia inhibitory factor. This method can culture female germline stem cells, but will not induce them to further differentiate into oocytes.

The female germline stem cells may be derived from follicular aspirates, which are often waste products in assisted reproduction. For example, a method for obtaining female germline stem cells comprises: (1) isolating ovarian tissue or cell clusters from follicular aspirates and performing cell dispersion to obtain single cells; (2) inoculating the cells obtained in step (1) onto inactivated feeder layer cells for culture and isolating female germline stem cells from the cultured cells.

### Ovarian Somatic-like Cells

In the present disclosure, the ovarian somatic-like cells comprise ovarian somatic-like cells obtained from the in vitro differentiation of human pluripotent stem cells. The human pluripotent stem cells comprise human induced pluripotent stem cells (human iPSCs) and human embryonic stem cells.

The embryonic stem cells or pluripotent stem cells involved in the present disclosure can be purchased through commercial channels, and embryonic stem cells or pluripotent stem cells may not necessarily be required by destroying embryos. As early as 1998, human embryonic stem cells and embryonic germ cells had been successfully established. For example, in 1998, the team led by Thomson finally established 5 human embryonic stem cell lines from 14 blastocysts: H1, H13, H14, H7 and H9; the team led by Gearhart isolated primitive stem cells from the gonadal ridges and mesentery of aborted fetuses aged 5-9 weeks, in order to avoid the ethical troubles caused by the direct use of embryos. See Chao Lan et al., "Research Progress of Human Embryonic Stem Cells", Advances in Modern Obstetrics and Gynecology, Volume 12, No. 4, July 2003. Based on the above work, in February 2000, the Wisconsin Alumni Research Foundation (WARF) established WiCell, an unofficial, non-profit subsidiary providing low-cost access to human embryonic stem cells for qualified scientists. There are many other institutions that provide ready-made human embryonic stem cells.

The ovarian somatic-like cells comprise ovarian somatic-like cells obtained from the in vitro differentiation of human pluripotent stem cells; preferably, the culture of the ovarian somatic-like cells comprises: (1) cell pre-culture; (2) treating the cells from (1) with a GSK3 inhibitor; (3) treating the cells from (2) with B/A/W, wherein the B/A/W is BMP4, Activin A and Wnt3a; (4) treating the cells from (3) with Follistatin, BMP4 and serum; (5) treating the cells from (4) with Follistatin and BMP4 to obtain a culture comprising human ovarian somatic-like cells.

In one or more embodiments, the "treatment" comprises "induction" or "induced treatment".

In one or more embodiments, the method is an in vitro/ex vivo culture method.

In one or more embodiments, after step (3), the obtained cells express mesendoderm markers such as brachyury, mixll and pax2.

In one or more embodiments, the human stem cells comprise human induced pluripotent stem cells (for example human induced pluripotent stem cells) and human totipotent stem cells (for example human embryonic stem cells).

In one or more embodiments, in the "ovarian somatic cell-like cells", the "somatic cell-like cells" refer to cells expressing Fox12, Cyp19a1 and Ptch1.

In one or more embodiments, cells are cultured by suspension culture in steps (1) to (3) and adherent culture in steps (4) to (5).

In one or more embodiments, in step (1), cells are cultured in a basal medium; in steps (2) to (5), cells are cultured in a basal medium added with GSK3 inhibitor, B/A/W, Follistain, BMP4 and serum; preferably, the basal medium is a stem cell medium (a culture medium suitable for culturing stem cells); more preferably, the basal culture medium is E8, mTesr or DMEM/F12 basal culture medium; more preferably, the basal culture medium is a basal culture medium added with 20±10% KSR, 1±0.5% NEAA, 1±0.5% Glutamax, 0.7±0.2 β-mercaptoethanol and 4-10ng/ml bFGF.

In one or more embodiments, in step (1), the time of cell pre-culture is 1 to 4 days; preferably 1.5 to 3 days; more preferably 1.5 to 2.5 days (for example 2 days).

In one or more embodiments, the culture medium for suspension culture is a liquid culture medium (culture solution). The culture medium for adherent culture is a corresponding liquid culture medium (culture solution).

In one or more embodiments, during suspension culture, low-adhesion culture dishes are used for culture.

In one or more embodiments, during adherent culture, the cells are attached to a culture dish treated with gelatin; preferably, the dish is treated with 0.2% gelatin.

In one or more embodiments, E8 is Essential 8^{™} medium from GIBCO, and mTeSR1 is a medium from Stemcell, Inc. Both culture media can be used for culturing pluripotent stem cells.

In one or more embodiments, in step (2), the GSK3 inhibitor comprises the group selected from: CHIR99021, LiCl, BIO or Ly2090314; preferably CHIR99021; preferably the concentration of CHIR99021 is 3 to 8 uM (such as 4, 4.5, 5, 5.5, 6 or 7 uM), more preferably 4 to 6 uM.

In one or more embodiments, in step (2), the culture is lasted for 30 to 48 hours (such as 32, 34, 36, 38, 40, 42 or 44 hours).

In one or more embodiments, in step (3), the concentration of BMP4 is 4-15 ng/ml (such as 5, 6, 7, 8, 9, 10, 11, 12, 13 or 14 ng/ml), preferably 9-12 ng/ml.

In one or more embodiments, the concentration of Activin A is 5-15 ng/ml (such as 6, 7, 8, 9, 10, 11, 12, 13 or 14 ng/ml), preferably 9-12 ng/ml.

In one or more embodiments, the concentration of Wnt3a is 5-15 ng/ml (such as 6, 7, 8, 9, 10, 11, 12, 13 or 14 ng/ml), preferably 9-12 ng/ml.

In one or more embodiments, in step (3), the culture is lasted for 2 to 9 days (such as 2.5, 3, 4, 5, 6, 7 or 8 days), preferably 2.5 to 7 days; more preferably 3 to 6 days.

In one or more embodiments, in step (4), the concentration of Follistain is 15-40 ng/ml (such as 18, 20, 22, 25, 28, 30, 32 or 35 ng/ml), preferably 20-30 ng/ml, more preferably 22-28 ng/ml.

In one or more embodiments, in step (4), the concentration of BMP4 is 4-15 ng/ml (such as 5, 6, 7, 8, 9, 10, 11, 12, 13 or 14 ng/ml), preferably 9-12 ng/ml.

In one or more embodiments, in step (4), the concentration of serum is 3-10% (v/v) (preferably 4-8%, such as 5%, 6%, 7% (v/v)).

In one or more embodiments, in step (4), the culture is lasted for 3 to 12 hours (such as 4, 5, 6, 7, 8, 9, 10 or 11 hours), preferably 4 to 10 hours; more preferably 5 to 8 hours.

In one or more embodiments, in step (5), the concentration of Follistain is 15-40 ng/ml (such as 18, 20, 22, 25, 28, 30, 32 or 35 ng/ml), preferably 20-30 ng/ml, more preferably 22-28 ng/ml.

In one or more embodiments, in step (5), the concentration of BMP4 is 4-15 ng/ml (such as 5, 6, 7, 8, 9, 10, 11, 12, 13 or 14 ng/ml), preferably 9-12 ng/ml.

In one or more embodiments, in step (5), the culture is lasted for 4 to 12 days (such as 4.5, 5, 6, 7, 8, 9, 10 or 11 days), preferably 5 to 10 days; more preferably 6 to 8 days.

### Establishment of Ovarian Organoids

The present disclosure provides a method for differentiating human female germline stem cells into mature oocytes in vitro. The method first involves co-culturing human female germline stem cells with ovarian somatic-like cells to establish a human ovarian organoid system, wherein follicles are generated within the human ovarian organoid system; subsequently, follicles are isolated from the human ovarian organoids and cultured in vitro to obtain mature oocytes. In a preferred embodiment, the method of the present disclosure comprises: (i) utilizing human female germline stem cells and ovarian somatic-like cells obtained from the in vitro differentiation of human induced pluripotent stem cells (iPSCs) for 3D co-culture to establish a human ovarian organoid system; (ii) isolating follicles from the ovarian organoids and performing 3D culture to obtain mature oocytes after in vitro maturation; further optionally, performing (iii) in vitro fertilization of the obtained mature oocytes ultimately developing them to the blastocyst stage.

In the present disclosure, the human female germline stem cells and ovarian somatic cell-like cells are separated to obtain female germline stem cells with positive markers (see PCT/CN2016/073429). The human female germline stem cells and ovarian somatic-like cells are mixed at a ratio of 1:(100-10000), preferably 1:(200-8000), more preferably 1:(300-6000). Thereby, the human female germline stem cells can receive adequate support from the ovarian somatic-like cells and undergo differentiation more effectively. In the most preferable embodiment, the human female germline stem cells and ovarian somatic-like cells are mixed at a ratio of approximately 1:1000.

In the present disclosure, a first organoid culture medium is applied, wherein some active components with recommended or preferable concentrations of this medium are listed in Table 1.

**Table 1**

| Components | Concentration | Preferable concentration | Examples |
|---|---|---|---|
| Serum or SPS serum substitute | 5-15%(v/v) | 8-12%(v/v) | 6, 7, 9, 10, 11, 13, 14%(v/v) |
| Retinoic acid | 0.8-3µM | 1-2µM | 0.9, 1.2, 1.5, 1.6, 1.8, 2.2, 2.5 µM |
| L-glutamine | 1-5 mM | 1.5-4mM | 1.5, 2, 2.5, 3, 3.5, 4, 4.5 mM |
| Non-essential amino acids | 0.5-2 mM | 0.8-1.2 mM | 0.6, 0.8, 1, 1.3, 1.5, 1.8 mM |
| spermidine | 50-150µM | 80-120µM | 60, 70, 90, 100, 110, 130, 140µM |
| sodium pyruvate | 15-50 mg/ml | 20-40 mg/ml | 18, 25, 30, 35, 42, 45 mg/ml |
| β-mercaptoethanol | 50-150 mM | 80-120 mM | 60, 70, 90, 100, 110, 130, 140 mM |
| EGF | 10-35 ng/mL | 15-25 ng/mL | 12, 16, 18, 20, 24, 28, 30 ng/mL |
| FGF | 5-15 ng/mL | 8-12 ng/mL | 6, 7, 9, 10, 11, 13, 14 ng/mL |
| Glial cell derived neurotrophic factor | 5-15 ng/mL | 8-12 ng/mL | 6, 7, 9, 10, 11, 13, 14 ng/mL |
| Leukemia inhibitory factor | 5-15 ng/mL | 8-12 ng/mL | 6, 7, 9, 10, 11, 13, 14 ng/mL |

In a preferred embodiment, the first organoid culture medium comprises MEMα or a similar culture medium (such as a culture medium of other MEM series) as the basal culture medium for providing nutrients suitable for cell growth. Preferably, this medium is supplemented with an SPS serum substitute instead of serum for easier conduction to the formation of cell aggregates.

After culture with the aforementioned medium, a second organoid culture medium is applied. Some active components with recommended or preferable concentrations of this medium are listed in Table 2, wherein bFGF, insulin, spermidine, BMP4 play particularly major roles.

**Table 2**

| Components | Concentration | Preferable concentration | Examples |
|---|---|---|---|
| Serum or SPS serum substitute | 1-5% | 1.5-4.5% | 1.5%, 2%, 2.5%, 3%, 3.5%, 4% or 4.5% |
| L-glutamine | 1-5 mM | 1.5~4mM | 1.5, 2, 2.5, 3, 3.5, 4, 4.5 mM |
| spermidine | 50-200µM | 80-150µM | 60, 70, 90, 100, 110, 130, 160µM |
| transferrin | 1-5 µg/mL | 1.5-4µg/mL | 1.5, 2, 2.5, 3, 3.5, 4, 4.5 mM |
| sodium selenite | 1-10 ng/mL | 3-6 ng/mL | 2, 4, 6, 8 ng/mL |
| Insulin | 5-15 ng/mL | 8-12 ng/mL | 6, 7, 9, 10, 11, 13, 14 ng/mL |
| FSH | 0.05-0.2IU/mL | 0.08-0.12IU/mL | 0.08, 0.1, 0.12, 0.14 IU/mL |
| ascorbic acid, | 80-400µM | 100-300µM | 90, 120, 150, 200, 250, 280, 350µM |
| EGF | 10-35ng/mL | 15-25ng/mL | 12, 16, 18, 20, 24, 28, 30 ng/mL |
| bFGF | 5-15 ng/mL | 8-12 ng/mL | 6, 7, 9, 10, 11, 13, 14 ng/mL |
| glial cell derived neurotrophic factor 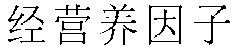 | 5-15 ng/mL | 8-12 ng/mL | 6, 7, 9, 10, 11, 13, 14 ng/mL |
| Leukemia inhibitory factor | 5-15 ng/mL | 8-12 ng/mL | 6, 7, 9, 10, 11, 13, 14 ng/mL |
| SCF | 5-15 ng/mL | 8-12 ng/mL | 6, 7, 9, 10, 11, 13, 14 ng/mL |
| BMP4 | 50-200 ng/mL | 80-150ng/mL | 60, 70, 90, 100, 110, 130, 160 ng/mL |
| β-mercaptoethanol | 25-75 mM | 40-60 mM | 30, 35, 45, 50, 55, 65, 70 mM |

In a preferred embodiment, the second organoid culture medium comprises MEMα or a similar culture medium (such as a culture medium of other MEM series) as the basal culture medium for providing nutrients suitable for cell growth. Compared to the first organoid culture medium, it further promotes the development of follicles in the organoids. Preferably, a small amount of serum is added to the culture medium for easier conduction to promote the development of follicles in the organoids.

After culture with the aforementioned medium, a third organoid culture medium is applied. Some active components of this medium and their recommended or preferred concentrations are listed in Table 3.

**Table 3**

| Components | Concentration | Preferable concentration | Examples |
|---|---|---|---|
| Serum or SPS serum substitute | 5-15%(v/v) | 8-12%(v/v) | 6, 7, 9, 10, 11, 13, 14%(v/v) |
| L-glutamine | 1-5 mM | 1.5-4mM | 1.5, 2, 2.5, 3, 3.5, 4, 4.5 mM |
| spermidine | 50-200µM | 80-150µM | 60, 70, 90, 100, 110, 130, 160µM |
| transferrin | 1-5 ng/mL | 1.5-4µg/mL | 1.5, 2, 2.5, 3, 3.5, 4, 4.5 mM |
| sodium selenite | 1-10 ng/mL | 3-6 ng/mL | 2, 4, 6, 8 ng/mL |
| Insulin | 5-15 ng/mL | 8-12 ng/mL | 6, 7, 9, 10, 11, 13, 14 ng/mL |
| FSH | 0.05-0.2IU/mL | 0.08-0.12IU/mL | 0.08, 0.1, 0.12, 0.14 IU/mL |
| ascorbic acid, | 80-400µM | 100-300µM | 90, 120, 150, 200, 250, 280, 350µM |
| EGF | 10-35ng/mL | 15-25ng/mL | 12, 16, 18, 20, 24, 28, 30 ng/mL |
| FGF | 5-15 ng/mL | 8-12 ng/mL | 6, 7, 9, 10, 11, 13, 14 ng/mL |
| glial cell derived neurotrophic factor 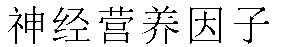 | 5-15 ng/mL | 8-12 ng/mL | 6, 7, 9, 10, 11, 13, 14 ng/mL |
| Leukemia inhibitory factor | 5-15 ng/mL | 8-12 ng/mL | 6, 7, 9, 10, 11, 13, 14 ng/mL |
| SCF | 5-15 ng/mL | 8-12 ng/mL | 6, 7, 9, 10, 11, 13, 14 ng/mL |
| BMP4 | 50-200 ng/mL | 80-150ng/mL | 60, 70, 90, 100, 110, 130, 160 ng/mL |
| β-mercaptoethanol | 25-75 mM | 40-60 mM | 30, 35, 45, 50, 55, 65, 70 mM |

In preferable embodiments, the third organoid culture medium comprises StemPro-34 as the basal medium. Unlike the previous MEM medium, StemPro-34 can provide more suitable and sufficient nutrition for organoid growth. Preferably, about 10% serum is added to the culture medium for easier conduction to promote the development of follicles in the organoids.

In a preferable embodiment of the present disclosure, antibiotics are added to the organoid culture medium to avoid contamination. Preferably, the antibiotics comprise penicillin and/or streptomycin.

In a preferable embodiment of the present disclosure, when establishing organoids, a 3D culture method is used in combination with the organoid culture medium to provide favorable conditions for follicle generation.

### Acquisition of Mature Oocytes

Following the establishment of the human ovarian organoid system as described above, follicles are generated within the system. Subsequently, follicles are isolated from the human ovarian organoids and cultured in vitro to obtain mature oocytes.

In the present disclosure, a first follicle culture medium is applied. Some active components with recommended or preferable concentrations of this medium are listed in Table 4, wherein FSH and PVP play particularly major roles.

**Table 4**

| Components | Concentration | Preferable concentration | Examples |
|---|---|---|---|
| Serum or SPS serum substitute | 3-8%(v/v) | 4-7%(v/v) | 5, 6, 7%(v/v) |
| L-glutamine | 1-5 mM | 1.5-4mM | 1.5, 2, 2.5, 3, 3.5, 4, 4.5 mM |
| transferrin | 1-5 µg/mL | 1.5-4µg/mL | 1.5, 2, 2.5, 3, 3.5, 4, 4.5 mM |
| sodium selenite | 1-10 ng/mL | 3-6 ng/mL | 2, 4, 6, 8 ng/mL |
| ascorbic acid, | 80-400µM | 100-300µM | 90, 120, 150, 180, 200, 250, 280, 350µM |
| EGF | 10-35ng/mL | 15-25ng/mL | 12, 16, 18, 20, 24, 28, 30 ng/mL |
| Activin-A | 50-200 ng/mL | 80-150ng/mL | 60, 70, 90, 100, 110, 130, 160 ng/mL |
| FSH | 0.05-0.15 IU/ml | 0.08-0.12 IU/ml | 0.06, 0.07, 0.09, 0.1, 0.11, 0.13, 0.14 IU/ml |
| β-mercaptoethanol | 25-75 mM | 40-60 mM | 30, 35, 45, 50, 55, 65, 70 mM |

In a preferable embodiment, the first follicle culture medium comprises MEMα or a similar culture medium (such as a culture medium of other MEM series) as the basal culture medium.

After culture with the aforementioned medium, a second follicle culture medium is applied. Some active components of this medium and their recommended or preferred concentrations are listed in Table 5.

**Table 5**

| Components | Concentration | Preferable concentration | Examples |
|---|---|---|---|
| Serum or SPS serum substitute | 5-15%(v/v) | 8-12%(v/v) | 6, 7, 9, 10, 11, 13, 14%(v/v) |
| L-glutamine | 1-5 mM | 1.5-4mM | 1.5, 2, 2.5, 3, 3.5, 4, 4.5 mM |
| sodium pyruvate | 1-5 mM | 1.5-4mM | 1.5, 2, 2.5, 3, 3.5, 4, 4.5 mM |
| sodium selenite | 2-10 ng/ml | 3-8 ng/ml | 2.5, 3.5, 4, 5, 6, 7, 9 ng/ml |
| bFGF | 5-15 ng/mL | 8-12 ng/mL | 6, 7, 9, 10, 11, 13, 14 ng/mL |
| EGF | 10-35ng/mL | 15-25ng/mL | 12, 16, 18, 20, 24, 28, 30 ng/mL |
| transferrin | 2-10 µg/ml | 3-8 µg/ml | 2.5, 3.5, 4, 5, 6, 7, 9 µg/ml |
| Insulin | 5-15µg/mL | 8-12 µg/mL | 6, 7, 9, 10, 11, 13, 14 µg/mL |
| PVP | 1-3.5%(w/v) | 1.5-2.5%(w/v) | 1.2, 1.6, 1.8, 2.0, 2.4, 2.8, 3%(w/v) |
| bmp15 | 8-30 ng/ml | 10-20 ng/ml | 9, 12, 15, 16, 18, 22, 25 ng/ml |
| gdf9 | 8-30 ng/ml | 10-20 ng/ml | 9, 12, 15, 16, 18, 22, 25 ng/ml |
| cAMP | 50-200 nM | 80-150 nM | 60, 70, 90, 100, 110, 130, 160 nM |
| FSH | 0.5-2 IU/ml | 0.8-1.2 IU/ml | 0.6, 0.8, 1, 1.3, 1.5, 1.8 IU/ml |
| LH | 0.5-2 IU/ml | 0.8-1.2 IU/ml | 0.6, 0.8, 1, 1.3, 1.5, 1.8 IU/ml |
| Estrogen | 0.5-2 ng/ml | 0.8-1.2 ng/ml | 0.6, 0.8, 1, 1.3, 1.5, 1.8 ng/ml |
| Progestrone | 0.5-2 ng/ml | 0.8-1.2 ng/ml | 0.6, 0.8, 1, 1.3, 1.5, 1.8 ng/ml |
| β-mercaptoethanol | 25-75 mM | 40-60 mM | 30, 35, 45, 50, 55, 65, 70 mM |

In a preferable embodiment, the second follicle culture medium comprises MEMα or a similar culture medium (such as a culture medium of other MEM series) as the basal culture medium.

After culture with the aforementioned medium, a third follicle culture medium is applied, wherein the third follicle culture medium is based on the second follicle culture medium with the addition of inactivated human follicular fluid. Preferably, the inactivated human follicular fluid can be added at 5-15% (v/v); more preferably at 8-12% (v/v); for example, at 6, 7, 9, 10, 11, 13, or 14 % (v/v). The addition of human follicular fluid at this stage is beneficial for later follicle development and the formation of antral follicles.

In a preferable embodiment of the present disclosure, antibiotics are added to the follicle culture medium to avoid contamination. Preferably, the antibiotics comprise penicillin and/or streptomycin.

In a preferable embodiment of the present disclosure, when establishing organoids, a 3D culture method is used in combination with the organoid culture medium to provide favorable conditions for follicle generation.

### Oocytes

Based on the novel findings, the present disclosure also provides a human oocyte product obtained by the method of the present disclosure, including human mature oocytes.

The present invention provides a human mature oocyte, wherein it is obtained by using human female germline stem cells as starting cells, mixing them with ovarian somatic-like cells to establish a human ovarian organoid system, and isolating it from the system, characterized by the following feature: extrusion of the first polar body.

Once the oocytes are mature, ICSI fertilization is performed and the appearance of pronuclei is observed after 16-24 hours. The appearance of pronuclei and bipolar bodies is regarded as a sign of fertilization. After fertilization, embryo culture medium is used for continued culture until development to the blastocyst stage.

The method and product of the present disclosure can not only provide an excellent model for studying the molecular mechanism of human ovarian somatic cell occurrence, but also provide a new approach for the clinical application of human ovarian somatic cells, and can be applied to assisted reproduction.

### Culture medium/kit

The present disclosure also provides medium for induction culture at various stages, including the aforementioned first organoid culture medium, the second organoid culture medium, the third organoid culture media; the first follicle culture medium, the second follicle culture medium and the third follicle culture medium.

In addition to the specific cytokines or chemical components listed in the embodiments of the present disclosure, cytokines or chemical components known in the art that have the same or similar functions as them can also be applied to the present disclosure. The analogs, proteins with the same functions (such as proteins with the same functions of growth factors) or compounds of the specifically listed components, equivalent compounds, analogs, derivatives and/or their salts, hydrates or precursors that induce the same target can also be used to replace the above-mentioned specific components to achieve the same technical effects. These analogs, proteins with the same functions or compounds should also be included in the present disclosure. Analogs of compounds comprise, but are not limited to: isomers and racemates of compounds. Compounds possess one or more asymmetric centers. Thus, these compounds may exist as racemic mixtures, individual enantiomers, individual diastereoisomers, diastereomeric mixtures, cis or trans isomers. The "precursor of a compound" refers to a compound that can be converted into a compound of any of the above compounds, or a salt or solution composed of a compound of any of the above compounds in the culture medium after being applied or treated by an appropriate method.

As a preferred embodiment of the present disclosure, the culture medium may further be added with ingredients for preventing bacterial contamination of cell culture, such as contamination by Gram-positive and Gram-negative bacteria, such as some antibiotics.

The cell culture medium (basal culture medium) may be, for example, but not limited to: MEM, StemPro-34, DMEM, DMEM/F12, RPMI1640, Neuronal basal, Fischers, etc. It should be understood that those skilled in the art are familiar with the preparation or purchase of the basal cell culture medium. The preferred cell culture medium is provided in the examples of the present disclosure.

The present disclosure also provides a kit, comprising the aforementioned first organoid culture medium, the second organoid culture medium, the third organoid culture media; the first follicle culture medium, the second follicle culture medium and the third follicle culture medium. Preferably, the kit also comprises the starting cells: human female germline stem cells, ovarian somatic-like cells. Alternatively, it may also comprise pluripotent stem cells for generating ovarian somatic-like cells. Preferably, if necessary, the kit also comprises a culture medium/reagent for isolating and maintaining cells. Preferably, the kit also comprises an instruction for use, so as to facilitate the use of the kit by those skilled in the art in research or clinical application.

The technical solution of the present disclosure is suitable for starting from human female germline stem cells, co-culturing them with ovarian somatic-like cells, and ultimately forming mature oocytes. Due to species differences, the induction of human female germline stem cells is significantly distinct from that of murine germline stem cells, mainly in: (1) The human 3D organoid culture time is substantially prolonged compared to murine; murine organoid culture requires only about 3 weeks, whereas human organoid culture requires 7 days + 2 weeks + 4 weeks = 7 weeks; (2) Differences in culture conditions during 3D culture, for instance, the use of Matrigel-treated Transwells in the present disclosure, the utilization of three follicle culture medium and the preference for certain effective factors/reagents such as transferrin, insulin, cAMP, LH, estrogen, progesterone, inactivated human follicular fluid, etc., wherein these factors are relevant to human ovarian or follicle development; (3) The follicle culture time is significantly extended; the time of murine culture is about 3-4 weeks, whereas the time of human culture is approximately 6 weeks.

To date, the field has not successfully established a protocol for a human female germline stem cell-derived ovarian organoid system that efficiently differentiates female germline stem cells into functional oocytes in vitro. The present disclosure, however, successfully obtains mature oocytes capable of producing healthy offspring via in vitro fertilization.

When applying for patents, it is recommended to emphasize the species origin and the specificity of the starting cells and to provide comprehensive activity validation data for the obtained functional oocytes. On this basis, emphasize: the first construction of human 3D ovarian organoids using human female germline stem cells and iPSC-induced differentiated ovarian somatic-like cells; and the first in vitro differentiation of human female germline stem cells into functional oocytes.

### Advantages of the present disclosure:

(1) The first construction of human 3D ovarian organoids using human female germline stem cells and iPSC-induced differentiated ovarian somatic-like cells.
(2) The first successful in vitro differentiation of human female germline stem cells into functional oocytes.

The disclosure is further described below in conjunction with specific embodiments. It should be understood that these examples are merely for illustrative purposes rather than intended to limit the scope of the disclosure. The experimental methods without specifying the specific conditions in the following examples generally used the conventional conditions, such as those described in J. Sambrook, Molecular Cloning: A Laboratory Manual (3rd ed. Science Press) or followed the manufacturer's recommendation.

### Example 1. Human pluripotent stem cells differentiating into human ovarian somatic cell-like cells

In this example, human ovarian somatic cell-like cells were prepared using human pluripotent stem cells as the basic cells.

### The first stage

Human induced pluripotent stem cells (iPSCs, obtained from Shanghai Oriental Hospital (Oriental Hospital affiliated to Tongji University) National Stem Cell Transformation Resource Bank) were suspended and cultured in low-adhesion culture bottles, with an inoculation volume of 5 x 10⁵ cells/bottle. DMEM, 20% KSR, 1% NEAA, 1% Glutamax, 0.7% β-mercaptoethanol (Sigma) were used as the basic culture medium. The culture conditions were: suspension culture at constant temperature of 37°C with 5% CO₂. The cells were cultured for 2 days to form embryoid bodies. The cells were placed for 5 minutes to allow the embryoid bodies to settle naturally. This part of the cell sphere can be used for subsequent culture.

### The second stage

Fresh culture medium was replaced for the second stage of culture. During this stage, the inventors successively adopted two culture methods. The agonist of WNT signaling pathway, GSK3 inhibitor CHIR99021, was added to the basal culture medium DMEM/F12 (comprising 20% KSR, 1% NEAA, 1% Glutamax, 0.7% β-mercaptoethanol) at an amount of 5 uM. The culture is lasted for 36 hours. The culture for suspension is at a constant temperature of 37°C and 5 %CO₂. The cells were placed to allow the cell sphere to settle naturally. This part of the cell sphere can be used for subsequent culture.

### The third stage

Wnt3a (with a final concentration of 6ng/ml), Activin A (with a final concentration of 6ng/ml) and BMP4 (with a final concentration of 10ng/ml) were added to the basal culture medium DMEM/F12 (comprising 20% KSR, 1% NEAA, 1% Glutamax and 0.7% β-mercaptoethanol) and treated for 3 days. The cells were placed to allow the cell sphere to settle naturally. This part of the cell sphere can be used for subsequent culture.

### The fourth stage

Fresh culture medium was replaced for the fourth stage of culture. Based on the previous step, the cells were attached to a culture dish treated with 0.2% gelatin; Follistain-like protein 1 (Follistain or Fstl1) (with a final concentration of 25 ng/ml), BMP4 (with a final concentration of 10 ng/ml), and FBS (with a final concentration of 5%) were added to the basal culture medium DMEM/F12 (comprising 20% KSR, 1% NEAA, 1% Glutamax, and 0.7% β-mercaptoethanol) for 5-8 h.

### The fifth stage

Fresh culture medium was replaced for the fifth stage of culture. Follistain (25 ng/ml) and BMP4 (10 ng/ml) were added to the basal medium DMEM/F12 (comprising 20% KSR, 1% NEAA, 1% Glutamax, and 0.7% β-mercaptoethanol) and treated for 8 days; no serum was added.

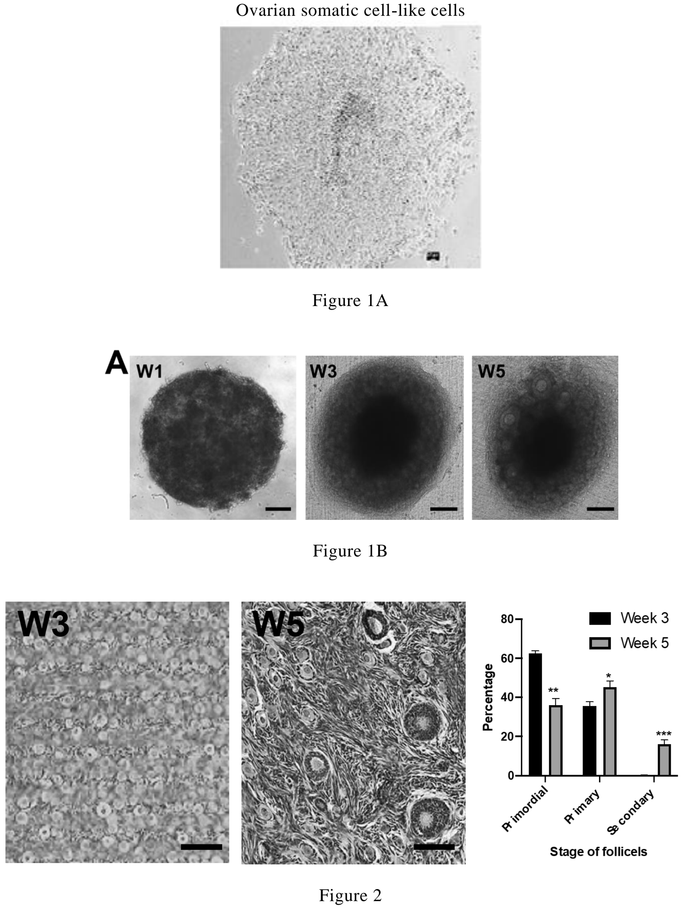

The bright field image of human ovarian somatic cell-like cells differentiated from human pluripotent stem cells obtained by the above method is shown in Figure 1A. It can be seen from the figure that the present disclosure obtains human ovarian somatic cell-like cells in an ideal state and in large quantities.

### Example 2. Establishment of Ovarian Organoids Based on Human FGSCs and Acquisition of Follicles

Human FGSCs were obtained from follicular aspirates and used to establish a co-culture system. The FGSCs were passaged approximately 10 times in vitro.

Ovarian somatic-like cells were obtained by in vitro induction and differentiation of iPSCs, as described in Example 1.

Human FGSCs and ovarian somatic-like cells were mixed at a ratio of 1:1000 in a 15 ml centrifuge tube and centrifuged at 1000 rpm for 5 min.

After centrifugation, the pellet was resuspended in organoid culture medium I (Table 6).

**Table 6**

| The basal medium MEMα (comprising 10% FBS (v/v)*) comprises the following components: | |
|---|---|
| retinoic acid | 1.5 µM |
| L-glutamine | 2 mM |
| non-essential amino acids | 1 mM |
| Spermidine | 100 µM |
| pyruvate | 30 mg/ml |
| β-mercaptoethanol | 100 mM |
| Human EGF | 20 ng/mL |
| Human bFGF | 10 ng/mL |
| human glial cell line-derived neurotrophic factor, GDNF | 10 ng/mL |
| human leukemia inhibitory factor, LIF | 10 ng/mL |
| penicillin | 30 mg/ml |
| streptomycin | 75 mg/ml |

| | |
|---|---|
| *10% FBS in MEMα can be replaced by 10% SPS (serum protein substitute). | |

Then, the suspension was mixed with Matrigel at a ratio of 1:1, and the cell suspension and Matrigel mixture was quickly added to a 96-well U-shaped plate, 5 µl per well. The plate was placed in a 37°C, 5% CO₂ incubator for 30 minutes to allow the Matrigel to solidify. The 96-well U-bottom plate was then removed, 100 µl of Organoid Culture Medium I was added to each well, and the U-bottom plate was placed in a 37°C, 5% CO₂ incubator for 7 days, with the medium being changed every 24 hours.

After 7 days of culture in the aforementioned 96-well U-bottom plate, the cell aggregates formed by the FGSCs and ovarian somatic-like cells were transferred to the upper chamber of a Transwell insert using a 1 ml pipette, with 6-10 aggregates cultured per Transwell. 1 ml of Organoid Culture Medium II (Table 7) was added to the upper chamber of the Transwell and 1.5 ml of Organoid Culture Medium II was added to the lower chamber.

The plate was placed in a 37°C, 5% CO₂ incubator. During culture, 1 ml of medium was aspirated from the lower chamber of the Transwell and replaced with 1 ml of fresh medium every 24 hours.

**Table 7**

| The basal medium MEMα (comprising 2% (v/v) fetal bovine serum (FBS)*) comprises the following components: | |
|---|---|
| L-glutamine | 2 mM |
| Spermidine | 100 µM |
| transferrin | 2.5 µg/ml |
| selenium | 4 ng/ml |
| human insulin | 10ng/ml |
| FSH | 0.1 IU/ml |
| ascorbic acid | 100 µM |
| Human EGF | 20 ng/mL |
| Human bFGF | 10 ng/mL |
| human glial cell line-derived neurotrophic factor | 10 ng/mL |
| human leukemia inhibitory factor | 10 ng/mL |
| SCF | 10ng/ml |
| BMP4 | 100ng/ml |
| β-mercaptoethanol | 50 mM |
| penicillin | 30 mg/ml |
| streptomycin | 75 mg/ml |

| | |
|---|---|
| *2% FBS in MEMα can be replaced by 2%SPS. | |

The organoids were cultured in organoid culture medium II for approximately 2 weeks, then the medium was changed to organoid culture medium III (Table 8). The organoids were cultured in organoid culture medium III for approximately 4 weeks or longer, during which 1 ml of medium was aspirated from the lower chamber of the Transwell and replaced with 1 ml of fresh medium every 24 hours.

**Table 8**

| The basal medium StemPro-34 SFM (comprising 10% FBS (v/v)*) comprises the following components: | |
|---|---|
| L-glutamine | 2 mM |
| Spermidine | 100 µM |
| transferrin | 2.5 µg/ml |
| selenium | 4 ng/ml |
| human insulin | 10ng/ml |
| FSH | 0.1 IU/ml |
| ascorbic acid | 100 µM |
| Human EGF | 20 ng/mL |
| Human bFGF | 10 ng/mL |
| human glial cell line-derived neurotrophic factor | 10 ng/mL |
| human leukemia inhibitory factor | 10 ng/mL |
| SCF | 10ng/ml |
| BMP4 | 100ng/ml |
| β-mercaptoethanol | 50 mM |
| penicillin | 30 mg/ml |
| streptomycin | 75 mg/ml |

| | |
|---|---|
| *10% FBS in StemPro-34 can be replaced by 10%SPS. | |

The inventors investigated the developmental characteristics of human ovarian organoid formation. In the third week of culture (W3), small follicles began to appear within the organoids. With continuous culture, the follicles grew, and by the fifth week of culture (W5), follicles composed of oocytes could be clearly observed (Figure 1B).

Histological section results also showed that the organoids contained follicles at different developmental stages (Figure 2).

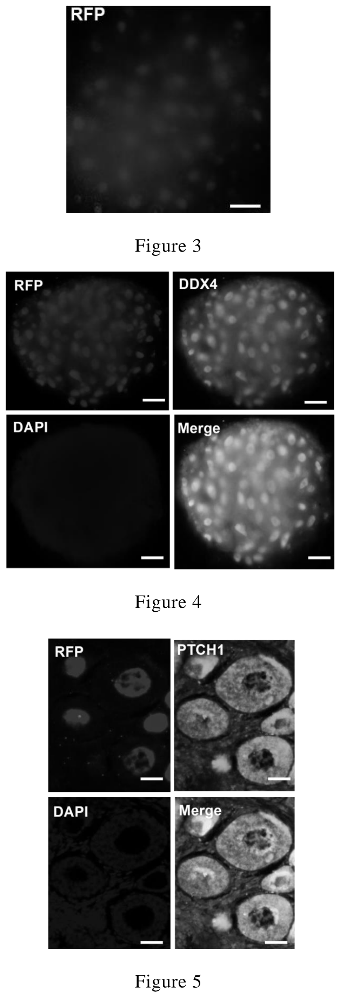

Significant RFP signal was detected within the ovarian organoids by fluorescence microscopy (Figure 3). This indicates that follicles formed within the ovarian organoids and were derived from the FGSCs.

Furthermore, tissue permeabilization of the ovarian organoids combined with immunohistochemistry confirmed that the oocytes within the follicles of the ovarian organoids were positive for the germ cell marker DDX4 (Figure 4).

Granulosa cells within the follicles of the ovarian organoids also expressed the granulosa cell marker PTCH1 (Figure 5).

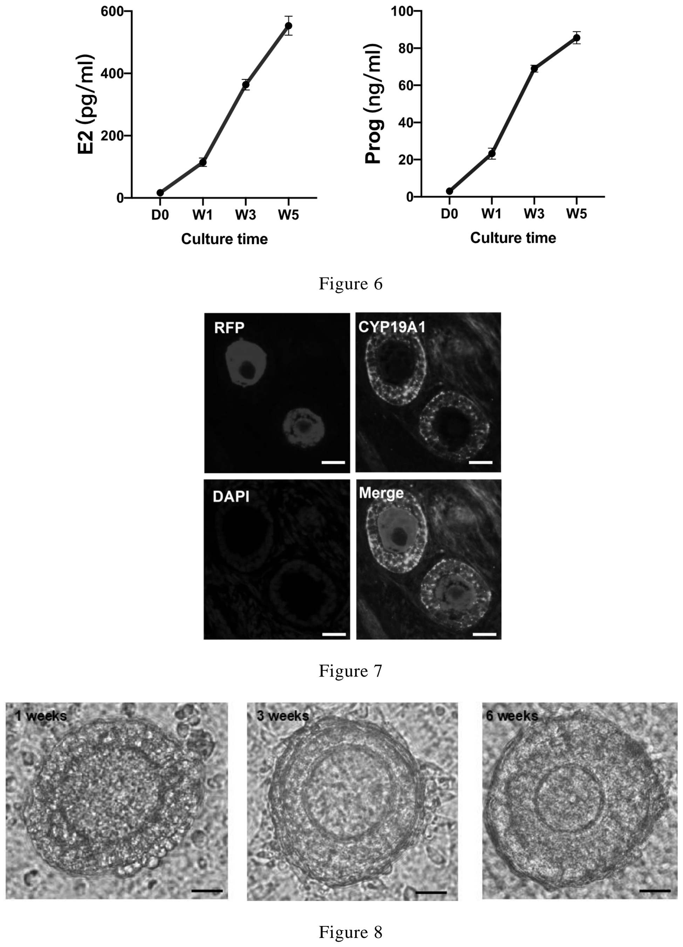

To detect the hormone secretion capacity of the ovarian organoids, the inventors measured the concentrations of progesterone and estradiol in the culture medium during organoid culture. The results showed that the concentrations of progesterone (prog) and estradiol (E2) continuously increased during ovarian organoid culture, indicating that the ovarian organoids possessed hormone secretion capability (Figure 6).

Tissue permeabilization of the ovarian organoids combined with immunohistochemistry confirmed that the granulosa cells within the follicles expressed CYP19A1, a marker associated with hormone secretion, further verifying the hormone secretion capacity of the ovarian organoids (Figure 7).

The above results indicate that ovarian organoids derived from human FGSCs contain follicles and possess hormone secretion function.

### Example 3. Acquisition of Mature Oocytes

A new Transwell was prepared by adding 1 ml of Matrigel to the upper chamber. The plate was placed in a 37°C, 5% CO₂ incubator for 30 minutes to allow the Matrigel to solidify. Individual follicles (from Example 2, cultured for 5-7 weeks) were carefully dissected using an insulin needle and transferred to the Matrigel-precoated upper chamber of the Transwell using a mouth pipette. The follicles were cultured in Follicle Culture Medium I (Table 9). During culture, 1 ml of medium was aspirated from the lower chamber of the Transwell and replaced with 1 ml of fresh medium every 24 hours.

**Table 9**

| The basal medium MEMα (comprising 5% (v/v) fetal bovine serum (FBS)*) comprises the following components: | |
|---|---|
| L-glutamine | 2 mM |
| transferrin | 2.5 µg/ml |
| selenium | 4 ng/ml |
| ascorbic acid | 100 µM |
| EGF | 20 ng/mL |
| Activin-A | 100ng/ml |
| FSH | 0.1 IU/ml |
| β-mercaptoethanol | 50 mM |
| penicillin | 30 mg/ml |
| streptomycin | 75 mg/ml |

| | |
|---|---|
| *5% FBS in MEMα can be replaced by 5%SPS. | |

After 1 week, the medium was changed to follicle culture medium II (Table 10). During culture, 1 ml of medium was aspirated from the lower chamber of the Transwell and replaced with 1 ml of fresh medium every 24 hours.

**Table 10**

| The basal medium MEMα (comprising 10% (v/v) fetal bovine serum (FBS)*) comprises the following components: | |
|---|---|
| L-glutamine | 2 mM |
| sodium pyruvate | 2mM |
| Sodium selenite | 5ng/ml |
| bFGF | 10 ng/mL |
| β-mercaptoethanol | 50 mM |
| EGF | 20 ng/mL |
| transferrin | 5 µg/ml |
| Insulin | 10 µg/ml |
| Polyvinylpyrrolidone (PVP) | 2% |
| bmp15 | 15ng/ml |
| gdf9 | 15ng/ml |
| cAMP | 100 nM |
| FSH | 1IU/ml |
| LH | 1IU/ml |
| Estrogen | 1 ng/ml |
| Progestrone | 1 ng/ml |
| penicillin | 30 mg/ml |
| streptomycin | 75 mg/ml |

| | |
|---|---|
| *10% FBS in MEMα can be replaced by 10%SPS. | |

After 2 weeks, the medium was changed to follicle culture medium III (follicle culture medium II supplemented with 10% (v/v) inactivated human follicular fluid) and cultured for approximately 3 weeks, during which 1 ml of medium was aspirated from the lower chamber of the Transwell and replaced with 1 ml of fresh medium every 24 hours. The inactivated human follicular fluid was obtained from a reproductive medicine center (with a role for promoting follicle growth).

During follicle culture, significant proliferation of peripheral granulosa cells and an increase in follicle diameter were observed (Figure 8).

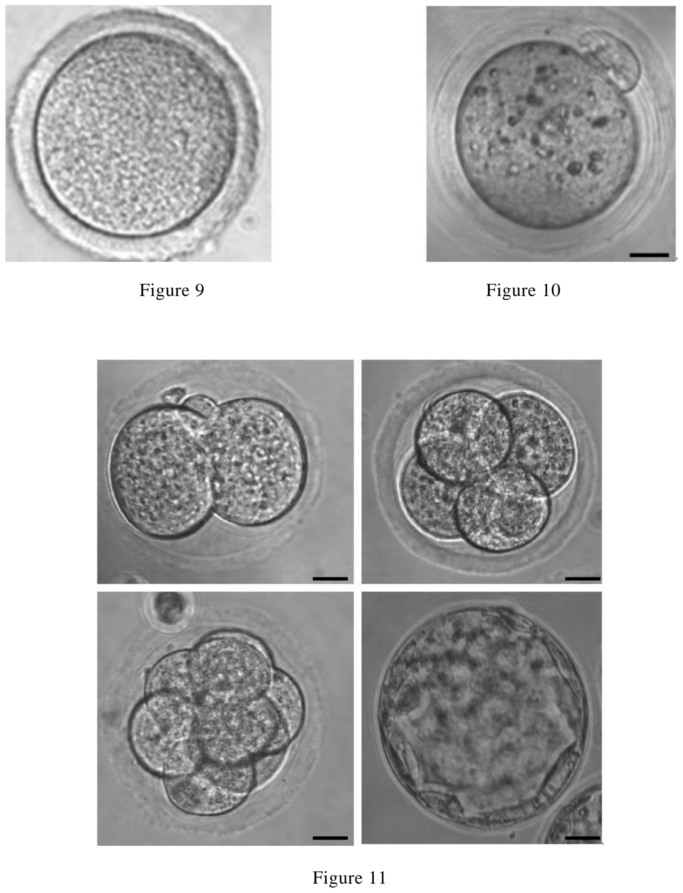

After 5 weeks of culture (counted from the start of isolated follicle culture), the oocytes within the follicles had developed to the Germinal Vesicle (GV) stage (Figure 9).

With continued culture, by the sixth week, both the oocytes and granulosa cells within the follicles were nearing maturity.

Immature follicles subjected to 3D culture were placed in in vitro maturation medium (basal maturation medium supplemented with 15% human follicular fluid) and cultured in a 37°C, 5% CO₂ incubator for 24-72h. Oocyte morphology and the degree of cumulus cell expansion were observed under a microscope, with the extrusion of the first polar body used as the marker for maturation. As shown in Figure 10, the first polar body was extruded, and the extrusion of the first polar body is a sign of oocyte maturation.

Once the oocytes are mature, ICSI fertilization is performed and the appearance of pronuclei is observed after 16-24 hours. The appearance of pronuclei and bipolar bodies is regarded as a sign of fertilization. After fertilization, embryo culture medium is used for continued culture until development to the blastocyst stage, as shown in Figure 11.

The above results demonstrate that the method of the present disclosure can differentiate and yield mature oocytes and the mature oocytes can subsequently achieve good growth and development.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. An in vitro method for preparing human mature oocytes, wherein the method comprises:
(1) co-culturing human female germline stem cells with ovarian somatic-like cells to establish a human ovarian organoid system, wherein follicles are generated within the human ovarian organoid system; and
(2) isolating follicles from the human ovarian organoids to obtain human mature oocytes by in vitro culture.

2. The method according to claim 1, wherein after obtaining the human mature oocytes, the method further comprises:
(3) performing in vitro fertilization of the human mature oocytes, followed by in vitro development.

3. The method according to claim 1, wherein, in step (1), the ovarian somatic-like cells comprise ovarian somatic-like cells obtained by in vitro differentiation of human pluripotent stem cells.

4. The method according to claim 3, wherein the ovarian somatic-like cells are cultured by the following steps comprising:
(1) cell pre-culture;
(2) treating the cells of (1) with a GSK3 inhibitor;
(3) treating the cells of (2) with B/A/W, wherein the B/A/W is BMP4, ActivinA and Wnt3a;
(4) treating the cells of (3) with Follistain, BMP4 and serum;
(5) treating the cells of (4) with Follistain and BMP4 to obtain a culture comprising human ovarian somatic cell-like cells.

5. The method according to claim 1, wherein, in step (1), the human female germline stem cells and ovarian somatic-like cells are mixed at a ratio of 1:(100-10000); preferably 1:(200-8000); more preferably 1:(300-6000).

6. The method according to claim 1, wherein after mixing the human female germline stem cells and ovarian somatic-like cells, the mixed cells are first cultured to form cell aggregates and then to form organoids; or
the human female germline stem cells are mixed with ovarian somatic cell-like cells and then cultured in three dimensions.

7. The method according to claim 5, wherein after mixing the human female germline stem cells and ovarian somatic-like cells, the mixture is cultured in a first organoid culture medium to form cell aggregates, comprising: retinoic acid, L-glutamine, spermidine, sodium pyruvate, β-mercaptoethanol, EGF, FGF, glial cell derived neurotrophic factor, leukemia inhibitory factor, non-essential amino acids; subsequently cultured in a second organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol; thereafter cultured in a third organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol.

8. The method according to claim 7, wherein the culture by the first organoid culture medium is lasted for 5-10 days, preferably 6-9 days; the culture by the second organoid culture medium is lasted for 10-20 days, preferably 12-18 days; or the culture by the third organoid culture medium is lasted for 18-45 days, preferably 20-40 days;
preferably, the first or second organoid culture medium comprises MEMα or a similar medium as the basal culture medium, wherein the first organoid culture medium comprises serum or a serum protein substitute, and the second organoid culture medium comprises serum or a serum protein substitute; the third organoid culture medium comprises StemPro-34 SFM as the basal culture medium and serum or a serum protein substitute.

9. The method according to claim 1, wherein, in step (2), the in vitro culture comprises: culturing in a first follicle culture medium comprising: L-glutamine, transferrin, sodium selenite, ascorbic acid, EGF, Activin-A, FSH, β-mercaptoethanol; subsequently culturing in a second follicle culture medium comprising: L-glutamine, sodium pyruvate, sodium selenite, bFGF, EGF, transferrin, Insulin, PVP, bmp15, gdf9, cAMP, FSH, LH, Estrogen, Progesterone, β-mercaptoethanol; thereafter culturing in a third follicle culture medium comprising: the second follicle culture medium and 5-20% (v/v) inactivated human follicular fluid.

10. The method according to claim 9, wherein the first follicle culture medium comprises MEMα or a similar medium as the basal culture medium and serum or a serum protein substitute; the second follicle culture medium comprises MEMα or a similar medium as the basal culture medium and serum or a serum protein substitute; preferably, the culture is three-dimensional culture.

11. A human mature oocyte prepared by the method according to any one of claims 1-10.

12. A use of human female germline stem cells and ovarian somatic-like cells for preparing human mature oocytes in vitro.

13. The use according to claim 12, wherein the human mature oocytes can be further developed in vitro into a blastocyst via in vitro fertilization.

14. A kit for preparing human mature oocytes in vitro, wherein it comprises:
(a) human female germline stem cells and ovarian somatic-like cells, or a mixture thereof;
(b) an organoid culture medium; and
(c) a follicle culture medium.

15. The kit according to claim 14, wherein, in (a), the human female germline stem cells and ovarian somatic-like cells are mixed at a ratio of 1:(100-10000); preferably 1:(200-8000); more preferably 1:(300-6000); or
(b) comprises: a first organoid culture medium comprising: retinoic acid, L-glutamine, spermidine, sodium pyruvate, β-mercaptoethanol, EGF, FGF, glial cell derived neurotrophic factor, leukemia inhibitory factor, non-essential amino acids; a second organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol; a third organoid culture medium comprising: spermidine, transferrin, sodium selenite, insulin, FSH, L-glutamine, ascorbic acid, EGF, bFGF, glial cell derived neurotrophic factor, leukemia inhibitory factor, SCF, BMP4, β-mercaptoethanol; preferably, the first or second organoid culture medium comprises MEMα or a similar medium as the basal culture medium, wherein the first organoid culture medium comprises serum or a serum protein substitute, and the second organoid culture medium comprises serum or a serum protein substitute; the third organoid culture medium comprises StemPro-34 SFM as the basal culture medium and serum or a serum protein substitute; or
(c) comprises: a first follicle culture medium comprising: L-glutamine, transferrin, sodium selenite, ascorbic acid, EGF, Activin-A, FSH, β-mercaptoethanol; a second follicle culture medium comprising: L-glutamine, sodium pyruvate, sodium selenite, bFGF, EGF, transferrin, Insulin, PVP, bmp15, gdf9, cAMP, FSH, LH, Estrogen, Progesterone, β-mercaptoethanol; a third follicle culture medium comprising: the second follicle culture medium and 5-20% (v/v) inactivated human follicular fluid.
